# EUROPEAN PATENT APPLICATION

(11) **EP 1 059 066 A1**
(43) Date of publication of application: **13.12.2000**
(21) Application number: 99905355.6
(22) Date of filing: 19.02.1999
(51) Int. Cl.: A61B 17/72, A61B 17/92

(54) **INTRAMEDULAR NAIL WITH SCREWS PRESSING THE NAIL AGAINST THE CORTICAL BONES, USED IN FRACTURES, PSEUDOARTHROSIS AND DEVIATIONS OF THE MECHANICAL AXIS, IN LONG BONES WHICH NOT SUBJECTED TO WALK**

(30) Priority: 20.02.1998 MX 9801405
(71) Applicant: Colchero Rozas, Fernando, Puebla, Puebla 72530 (MX)
(72) Inventor: Colchero Rozas, Fernando, Puebla, Puebla 72530 (MX)
(74) Representative: EGLI-EUROPEAN PATENT ATTORNEYS
(86) International application number: MX9900005
(87) International publication number: WO9942052

(57) **Abstract**

This invention relates to a new intramedular nail with screws pushing the nail against the bone corticals, to be used in fractures, pseudoarthrosis and mechanical axis deviations, on long, non subject to march bones; having the capability of maintain those ends of the bone to be bonded to each other, free of macro-movements, but admitting the micro-movements that stimulate said bond; with a very simple, easy to use manipulating device, to push, pull or rotate said nail being manipulated; thus facilitating the surgical procedure and reducing the damages caused on the soft tissues in the neighborhood of the wound under treatment. Said nail is characterized by comprising a manipulator comprising a sole piece with an end of greater diameter and shorter length than the opposite end; said greater diameter and shorter length end having an outer frusto-conical shape and the opposite end having an engraving to minimize the risk of hand slippery thereon.

## Description

### FIELD OF THE INVENTION

This invention relates to the so-called by orthopedists intramedular nails, usable in the reduction of fractures, and in the treatment of pseudoarthrosis, osteotomies and mechanical axis deviations, mainly with long, non subject to march, bones.

### BACKGROUND OF THE INVENTION

Prior to this invention, plasters, non-fastening intramedular nails and plates with screws positioned on the bone surface, have been used in the treatment of fractures, pseudoarthrosis, osteotomies, non-consolidated fractures, mechanical axis deviations, and the like. These procedures are prone to result in a lack of union in fractures, pseudoarthrosis or in deviations of the bone thus requiring additional surgery. Frequently, mainly with screwed plates, bone infections appear due to the injury produced on the bone periosteum (blood donor for the cortical). Said infections are feared by surgeons due to sequelae and the treatment difficulties.

The use of treatments for this type of wounds, by using solid or hollow nails, with a series of orifices along the longitudinal axis thereof, according to the state of art, also has a series of drawbacks that make them unsuitable for the treatment of this type of wounds in bones.

Due to their extremely strong fastening, nails attached by means of screws sustain rigidly the ends of a fracture and there is also a motion possibility without the risk of displacements of said ends; but also have the drawback that the micro-movements stimulating the bone union are also impaired.

During the procedure to place an intramedular nail in a bone, it is necessary to manipulate said nail by pushing and rotating the same in order to situate it in place. Due to the characteristics of the presently employed nails, additional devices are required to carry out said manipulation as far away from the patient body as possible. Said devices are relatively complicated and make the surgical procedures more difficult. Furthermore, due to the connection way of said nail to the manipulating devices said nails must be provided with outer threads; and the screwing operation near the body means a problem for the threaded attachment as well as for the prevention from the tissues to be contacted thereby, such as the bone marrow, the neighbor muscles and even the skin itself, can be damaged during said screwing operation.

### OBJECTS OF THE INVENTION

The present invention has as its first object to make possible an intramedular nail that allows for the treatment of fractures, pseudoarthrosis, etc., by maintaining free of macro-movements the ends of the bone to be connected to each other, but admitting those micro-movements capable of stimulating said connection.

It is a second object of the invention to provide a nail of this type, with the additional characteristic of comprising a manipulating device, very simple and easy to use in pushing, pulling or rotating the nail, thus facilitating the surgical procedure.

It is a third object of the invention to provide a nail the connection of which with its manipulating device minimizes the damages to the tissues immediate to the wound under treatment.

Other further objects and advantages of the invention will be obvious following the reading and understanding of this disclosure.

### SUMMARY OF THE INVENTION

Nails of the present invention are part of a system constituted by orthopedic nails and screws. Nails penetrate the medular cavity comprised by the types of bones already mentioned in the title of the invention. The stabilization of said wounds are obtained when said intramedular nail is pushed by said screws against the inner wall of the bone medular channel (corticals). Upon the pin being tightened by the screws against the corticals the movements (macro-movements) that prevent the fracture consolidation are impaired, but the micro-movements capable of activating the wounds, are permitted.

Nails of this invention are semi-cylindrical, with a plane surface where one of the ends of each screw will be fastened. Due to the shape and special characteristics of every bone, said nails and screws are to be adapted thereto with different thicknesses, lengths and even curvatures in one or both ends.

Due to the thickness of the different corticals and to the muscular forces acting on the bones, said screws are of different lengths and thicknesses.

Now an illustrative embodiment of the invention will be described for a better understanding of the invention; but in no way means a restrictive disclosure.

### BRIEF DESCRIPTION OF THE DRAWINGS

FIGURE 1 illustrates the manipulator for rotating and longitudinally displacing in one sense and in the opposite sense a nail, in order to set said nail in the wound o deviation under treatment.
FIGURE 2 shows a detail in a cross-sectional view taken following the line X-X' of FIGURE 1.
FIGURE 3 shows the screw that fastens the nail manipulator to said nail.
FIGURE 4 illustrates the nail of this invention showing the curvature that makes the nail capable of being withdrawn from the bone medular channel across the cortical, also illustrating the end where the manipulator is fastened and likewise the plane portion of the nail.
FIGURE 5 shows the detail of the end where the manipulator and the attaching screw are connected to each other.

### DETAILED DESCRIPTION OF THE INVENTION

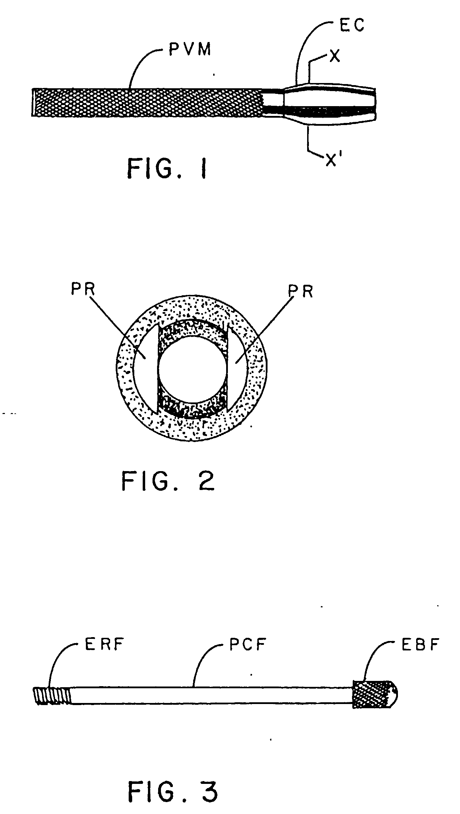

In FIGURE 1 a manipulator is shown with one end to be attached to a nail having an outer conical profile "EC" and a beveled end at the opposite end. The conical outer profile allows that this manipulator penetrates the soft tissues surrounding the bone at the zone where said manipulator is to be fastened to the nail. Due to the shape of this profile, upon said manipulator entering the tissues, opens them, without crushing nor cutting the same. Said manipulator beveled end "PVM" provides a better, slipping-free grip for the surgeon.

On the manipulator portion where an outer conical profile is formed, as can be seen in FIGURE 2, there are provided rectangular projections from the inner cylindrical surface. Thereat the outer rectangular recesses of the nail, at the end that remains in the exterior of the bone, are to be connected. Said manipulator comprises an interior longitudinal channel, of circular shape, through which a substantially elongated fastener as illustrated in FIGURE 3 is passed.

Said fastener has an externally threaded end, with a thread tip having a diameter same as the central portion of said fastener "PCF" which is completely flat. The outer diameter of the central portion and the threaded end of the fastener is slightly lower than the inner diameter of the longitudinal inner channel of the manipulator, in order the threaded end and the central portion of said fastener can easily slide within the inner channel of said manipulator. At the end opposite to the threaded end, said fastener has an outer stepped diameter, greater than the rest of said fastener, and with the surface thereof beveled, so as the surgeon is able to rotate said fastener to screw it on and out regarding said nail, without slippery risks.

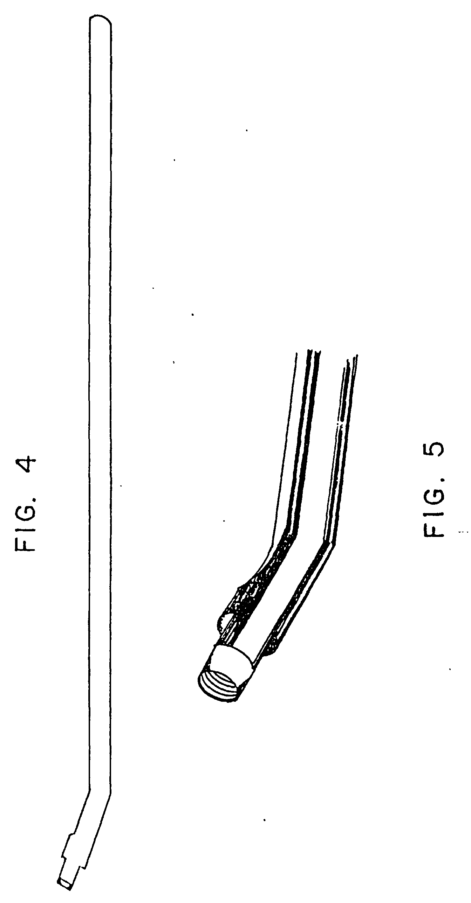

In FIGURE 4 said nail can be seen with its two straight portions at a different angle; the longer one is the portion to be housed in the medular channel of the bone under treatment, and the shorter one is that which will remain outside the bone, and to the end of which said manipulator is to be fastened. The transversal cross-section of said nail is generally semi-cylindrical, with a plane portion where the screws traversing the cortical of the bone under treatment, will contact; thus pushing said nail against the medular channel wall, on the portion contrary to the site where said nail enter.

The protruding end of the nail is shorter and is at a predetermined angle. At this end, on the peripheral portion, there is a recess mating said rectangular projections of the interior channel of said manipulator, at the end where the outer portion has a frusto-conical profile. Likewise, at the transversal face, this end of the nail bears a central internal thread, where the fastener will be screwed to said manipulator.

The details of the end of the nail protruding from the bone, can be seen in FIGURE 5; where both the recesses and the start of the interior thread are shown.

The use of the nail will be disclosed now. In the first place, said nail is fastened to the manipulator by means of the fastener. In order to situate it in place, an incision is made distal to the wound in the bone region where said nail is to be introduced, making the incision profound down to the bone. An opening is carried out at the bone cortical in this point, down to the medular channel. The nail is introduced into said medular channel beyond the two fragments (with image magnifier when is made through closed focus, or by opening the focus of the fracture when is made through open focus) by pushing, rotating and pulling said nail, by means of said manipulator. Soft tissues are opened at the point where the screws that fix the nail in both bone segments are to be introduced. Said screws are introduced by rotating said nail so as the ends of said screws make contact with the plane portion of said nail. Said nail is fastened and the wounds are closed, both those of the open focus, as the case may be, and those of the screws, as well as the incision made in order to introduce said nail. Once the nail fastened, and the manipulator complied with its task, this latter is unscrewed and withdrawn, whereby only a portion of the shorter end of said nail remains exposed.

The longitudinal dimensions of the longer portion of the nail will depend on the characteristics of the bone under treatment; when dealing with a long bone or one that requires greater support, a nail will be used with its longer portion of greater length than a nail for a shorter bone, or for one with no need to support substantial efforts.

The angle between the longer portion of the nail and the shorter portion thereof, will depend, in every application, on the place where the fracture appears, where the incision is to be made in order to introduce said nail, and the like.

Finally, the shorter portion will have also a variable length, depending on the thickness of the soft tissue surrounding the zone where said nail is to be introduced and on the region of the body where said zone is located.

In another embodiment of the invention, the fastening of the shorter end of the nail to said manipulator will be made by means of an inner fluting in said manipulator, mating outer flutings in said nail.

Other further specific embodiments of the invention will be apparent to those skilled in the art, from a consideration of this description of practical embodiments of the invention herein disclosed. It will be understood that said disclosure and specific embodiments will be considered only as illustrative, with the true scope and spirit of the invention being stated by the appended claims.

Having thus described the invention, it is considered as a novelty and therefore, it is claimed as a property the contents of the following claims.

## Claims

1. Intramedular nail with screws that push said nail against the bone corticals, to be used in fractures, pseudoarthrosis and mechanical axis deviations, in long, non subject to march bones; of the type lacking of orifices for the fastening thereof by means of screws, and includes a manipulator to push, rotate and pull said nail in the medular channel of the bone under treatment; with said nail having a longer portion and a shorter portion; characterized in that said nail is of semi-cylindrical cross-section, with a plane portion where said screws will push it against the bone cortical; with said manipulator being of a sole piece with an end of greater diameter and shorter length, than the opposite end; said greater diameter and shorter length end having an outer frusto-conical shape and the opposite end having an engraving in order to minimize the risk of slippery of a hand; said manipulator will be fastened to the shorter end of said nail by means of recesses in said nail or manipulator and projections in said manipulator or nail, respectively, and a fastener longitudinally traversing said manipulator and screwed on the inner thread of the transversal face of said shorter end.

2. Intramedular nail with screws that push said nail against the bone corticals, to be used in fractures, pseudoarthrosis and mechanical axis deviations, in long, non subject to march bones, as claimed in the preceding Claim, characterized in that said fastener of said manipulator comprises, at the end opposite to the thread, a portion with a diameter greater than the outer diameter of said manipulator, at the beveled end; this portion being the one where said fastener will be gripped to rotate the same and screw the same to said nail.
